# FASCICULE DE BREVET EUROPEEN

(11) **EP 2 346 911 B1**
(45) Date de publication et mention de la délivrance du brevet: **14.08.2013**
(21) Numéro de dépôt: 09768173.8
(22) Date de dépôt: 10.11.2009
(51) Int. Cl.: C08F 210/02, C08F 218/00, C08L 23/08, C08L 35/00, C09J 123/08, C09J 135/00, C08J 5/18

(54) **FABRICATION DE COPOLYMERES ETHYLENE/ESTER VINYLIQUE D'ACIDE CARBOXYLIQUE A PARTIR DE MATIERES RENOUVELABLES, COPOLYMERES OBTENUS ET UTILISATIONS**
HERSTELLUNG VON ETHYLEN-/CARBONSÄURE-VINYLESTERCOPOLYMEREN AUS ERNEUERBAREN MATERIALIEN, DARAUS GEWONNENE COPOLYMERE UND VERWENDUNG
MANUFACTURE OF ETHYLENE/CARBOXYLIC ACID VINYL ESTER COPOLYMERS FROM RENEWABLE MATERIALS, COPOLYMERS OBTAINED AND USES

(30) Priorité: 13.11.2008 FR 0857686
(43) Date de publication de la demande: 27.07.2011
(73) Titulaire: Arkema France, 92700 Colombes (FR)
(72) Inventeur: DEVISME, Samuel, F-76000 Rouen (FR); CHOPINEZ, Fabrice, PA 19904 (US); LAURICHESSE, Christian, F-64140 Lons (FR); ROUSSEL, Thomas, F-69006 Lyon (FR); DUBOIS, Jean-Luc, F-69390 Millery (FR)
(74) Mandataire: Gavin, Pablo
(86) Numéro de dépôt international: PCT/FR2009/052165
(87) Numéro de publication internationale: WO 2010/055257

(56) Documents cités:
- EP-A- 0 125 983
- FR-A- 2 884 817
- GB-A- 790 559
- GB-A- 1 219 960
- GB-A- 1 443 394
- US-A- 3 595 740
- US-A- 4 234 752
- US-A- 4 456 622
- US-A- 4 670 620
- US-A- 5 091 261
- US-A- 5 753 474
- US-A1- 2007 032 614

## Description

### Domaine de l'invention

La présente invention se rapporte à un procédé de fabrication de copolymères d'éthylène et d'au moins un ester vinylique dans lesquels l'éthylène est au moins en partie obtenu à partir de matières premières renouvelables.

### Etat de la technique

Un des problèmes posés par les copolymères comprenant de l'éthylène de l'art antérieur est qu'ils sont réalisés à partir de matières premières d'origine fossile (pétrole) non renouvelable. Or les ressources en pétrole sont limitées, l'extraction du pétrole requiert d'aller creuser de plus en plus profond et dans des conditions techniques toujours plus difficiles nécessitant des équipements sophistiqués et la mise en oeuvre de procédés toujours plus coûteux en énergie. Ces contraintes ont une conséquence directe sur le coût de fabrication de l'éthylène et donc des copolymères à base d'éthylène.

De manière avantageuse et surprenante, les inventeurs de la présente demande ont mis en oeuvre un procédé de fabrication industriel des copolymères à base d'éthylène à partir de matières premières renouvelables.

Le procédé selon l'invention permet de s'affranchir au moins en partie des matières premières d'origine fossile et de les remplacer par des matières premières renouvelables.

En outre les copolymères à base d'éthylène obtenus suivant le procédé selon l'invention sont de qualité telle qu'ils peuvent être utilisés dans toutes les applications dans lesquelles il est connu d'utiliser ces copolymères, y compris dans les applications les plus exigeantes.

### Résumé de l'invention

L'invention a pour objet un procédé de fabrication de copolymères d'éthylène et d'au moins un ester vinylique d'acide carboxylique dans lesquels l'éthylène est au moins en partie obtenu à partir de matières premières renouvelables.

Dans la présente demande, il est fait référence à des composés, comprenant des atomes de carbone, « obtenus à partir de matières premières renouvelables », ainsi au sens de la présente demande, on entendra que ces composés comprennent des atomes de carbone de ¹⁴C qui peuvent être déterminés selon la norme ASTM D 6866-06. Par exemple, le copolymère peut comporter au moins 0,24 10⁻¹⁰ % en masse de ¹⁴C.

Au sens de la présente demande, on entendra par « copolymères d'éthylène et d'au moins un ester vinylique » aussi bien les copolymères constitués de deux monomères que des terpolymères comprenant de l'éthylène et au moins un ester vinylique.

Avantageusement les esters vinyliques utilisés dans les copolymères selon la présente invention sont des esters vinyliques d'acides carboxyliques en C₂-C₈, de préférence ils sont choisis parmi les esters vinyliques d'acides carboxyliques en C₂-C₄ et de manière encore plus préférée ils sont choisis parmi l'acétate de vinyle et le propionate de vinyle.

Selon une variante particulière, au moins une partie des atomes de carbone de l'ester vinylique est d'origine renouvelable.

Les copolymères obtenus selon la présente invention peuvent également être des terpolymères d'éthylène, d'au moins un ester vinylique et d'au moins un anhydride d'acide carboxylique insaturé dans lesquels l'éthylène est au moins en partie obtenu à partir de matières premières renouvelables, et de manière optionnelle au moins une partie des atomes de carbone de l'ester vinylique et/ou au moins une partie des atomes de carbone de l'anhydride d'acide carboxylique insaturé sont d'origine renouvelable.

Ainsi, selon une première variante particulière de ces terpolymères, au moins une partie des atomes de carbone de l'ester vinylique est d'origine renouvelable.

Selon une deuxième variante particulière de ces terpolymères, au moins une partie des atomes de carbone de l'anhydride d'acide carboxylique insaturé est d'origine renouvelable.

Selon une troisième variante particulière de ces terpolymères, au moins une partie des atomes de carbone de l'ester vinylique est d'origine renouvelable et au moins une partie des atomes de carbone de l'anhydride d'acide carboxylique insaturé est d'origine renouvelable.

De préférence, l'ester vinylique est choisi parmi l'acétate de vinyle et le propionate de vinyle.

De préférence, l'anhydride d'acide carboxylique insaturé est l'anhydride maléique.

Ainsi, selon une première variante particulière de ces terpolymères, au moins une partie des atomes de carbone de l'ester vinylique est d'origine renouvelable.

Selon une deuxième variante particulière de ces terpolymères, au moins une partie des atomes de carbone de l'anhydride d'acide carboxylique insaturé est d'origine renouvelable.

Les copolymères obtenus selon la présente invention peuvent également être des terpolymères d'éthylène, d'au moins un ester vinylique tel que l'acétate de vinyle et d'au moins un ester d'acide carboxylique insaturé tel qu'un ester acrylique ou méthacrylique d'acide carboxylique dans lesquels l'éthylène est au moins en partie obtenu à partir de matières premières renouvelables, et de manière optionnelle au moins une partie des atomes de carbone de l'ester vinylique et/ou au moins une partie des atomes de carbone de l'ester d'acide carboxylique insaturé sont d'origine renouvelable.

La présente demande concerne également les mélanges de copolymères selon l'invention, les compositions comprenant ces copolymères et les utilisations de ces copolymères.

Les copolymères de l'éthylène et d'au moins un ester vinylique obtenus selon la présente demande sont susceptibles d'être obtenus selon le procédé de fabrication comprenant les étapes suivantes :
a) fermentation de matières premières renouvelables et, éventuellement purification pour produire au moins un alcool choisi parmi l'éthanol et les mélanges d'alcools comprenant de l'éthanol ;
b) déshydratation de l'alcool obtenu pour produire au moins un alcène choisi parmi l'éthylène et les mélanges d'alcènes comprenant de l'éthylène et, éventuellement purification de l'alcène pour obtenir de l'éthylène,
c) copolymérisation de l'éthylène avec au moins un comonomère choisi parmi les esters vinyliques d'acides carboxyliques,
d) isolation du copolymère obtenu.

Les terpolymères d'éthylène, d'au moins un ester vinylique et d'au moins un anhydride d'acide carboxylique insaturé selon la présente demande sont susceptibles d'être obtenus selon le procédé de fabrication décrit ci-dessus dans lequel l'étape c) est une étape de copolymérisation de l'éthylène avec au moins un ester vinylique et au moins un anhydride d'acide carboxylique insaturé.

D'autres objets, aspects, caractéristiques de l'invention apparaîtront à la lecture de la description suivante.

### Description détaillée de l'invention

L'étape a) du procédé de fabrication de copolymères de l'éthylène et d'au moins un ester vinylique selon l'invention comprend la fermentation de matières premières renouvelables pour produire au moins un alcool, ledit alcool étant choisi parmi l'éthanol et les mélanges d'alcools comprenant de l'éthanol.

Une matière première renouvelable est une ressource naturelle, par exemple animale ou végétale, dont le stock peut se reconstituer sur une période courte à l'échelle humaine. Il faut en particulier que ce stock puisse se renouveler aussi vite qu'il est consommé. Par exemple, les matières végétales présentent l'avantage de pouvoir être cultivées sans que leur consommation aboutisse à une diminution apparente des ressources naturelles.

A la différence des matériaux issus de matières fossiles, les matières premières renouvelables contiennent du ¹⁴C. Tous les échantillons de carbone tirés d'organismes vivants (animaux ou végétaux) sont en fait un mélange de 3 isotopes : ¹²C (représentant environ 98,892 %), ¹³C (environ 1,108 %) et ¹⁴C (traces: 1,2.10⁻¹⁰ %). Le rapport ¹⁴C/¹²C des tissus vivants est identique à celui de l'atmosphère. Dans l'environnement, le ¹⁴C existe sous deux formes prépondérantes : sous forme de gaz carbonique (CO₂), et sous forme organique, c'est-à-dire de carbone intégré dans des molécules organiques.

Dans un organisme vivant, le rapport ¹⁴C/¹²C est maintenu constant par le métabolisme car le carbone est continuellement échangé avec l'environnement extérieur. La proportion de ¹⁴C étant constante dans l'atmosphère, il en est de même dans l'organisme, tant qu'il est vivant, puisqu'il absorbe ce ¹⁴C au même titre que le ¹²C ambiant. Le rapport moyen de ¹⁴C/¹²C est égal à 1,2x10⁻¹².

Le ¹²C est stable, c'est-à-dire que le nombre d'atomes de ¹²C dans un échantillon donné est constant au cours du temps. Le ¹⁴C est radioactif, le nombre d'atomes de ¹⁴C dans un échantillon décroît au cours du temps (t), sa demi-vie étant égale à 5730 ans.

La teneur en ¹⁴C est sensiblement constante depuis l'extraction des matières premières renouvelables, jusqu'à la fabrication du copolymère selon l'invention selon l'invention et même jusqu'à la fin de vie de l'objet fabriqué en ledit copolymère.

Par conséquent, la présence de ¹⁴C dans un matériau, et ce, quelle qu'en soit la quantité, donne une indication sur l'origine des molécules le constituant, à savoir qu'elles proviennent de matières premières renouvelables et non de matériaux fossiles.

La quantité de ¹⁴C dans un matériau peut être déterminée par l'une des méthodes décrites dans la norme ASTM D6866-06 (Standard Test Methods for Determining the Biobased Content of Natural Range Materials Using Radiocarbon and Isotope Ratio Mass Spectrometry Analysis).

Cette norme comporte trois méthodes de mesure du carbone organique issu de matières premières renouvelables, dénommé en langue anglaise « biobased carbon ». Les proportions indiquées pour le copolymère de l'invention sont de préférence mesurées selon la méthode par spectrométrie de masse ou la méthode par spectrométrie à scintillation liquide décrite dans cette norme, et tout préférentiellement par spectrométrie de masse.

Ces méthodes de mesure évaluent le rapport des isotopes ¹⁴C/¹²C dans l'échantillon et le comparent à un rapport des isotopes ¹⁴C/¹²C dans un matériau d'origine biologique donnant le 100% standard, afin de mesurer le pourcentage de carbone organique de l'échantillon.

De préférence, le copolymère selon l'invention comprend une quantité de carbone issu de matières premières renouvelables supérieure à 20%, de préférence supérieure à 50% en masse par rapport à la masse totale de carbone du copolymère.

En d'autres termes, le copolymère peut comporter au moins 0,24 10⁻¹⁰ % en masse de ¹⁴C, et de préférence au moins 0,6 10⁻¹⁰ % en masse ¹⁴C.

Avantageusement la quantité de carbone issue de matières premières renouvelables est supérieure à 75%, de préférence égale à 100% en masse par rapport à la masse totale de carbone du copolymère.

En tant que matières premières renouvelables, on pourra utiliser des matières végétales ; des matières d'origine animale ou des matières d'origine végétale ou animale issues de matériaux récupérés (matériaux recyclés).

Au sens de l'invention, les matières d'origine végétale contiennent au moins des sucres et/ou amidons.

Les matières végétales contenant des sucres sont essentiellement la canne à sucre et la betterave sucrière, on peut également citer l'érable, le palmier-dattier, le palmier à sucre, le sorgho, l'agave américain ; les matières végétales contenant des amidons sont essentiellement les céréales et légumineuses comme le maïs, le blé, l'orge, le sorgho, le froment, le riz, la pomme de terre, le manioc, la patate douce, ou encore les algues.

Parmi les matières issues de matériaux récupérés, on peut notamment citer les déchets végétaux ou organiques comprenant des sucres et/ou amidons.

De préférence les matières premières renouvelables sont des matières végétales.

La fermentation des matières renouvelables s'effectue en présence d'un ou plusieurs microorganismes adéquats, ce microorganisme peut éventuellement avoir été modifié naturellement par une contrainte chimique ou physique, ou génétiquement on parle alors de mutant. Classiquement le microorganisme utilisé est *Saccharomyces cerevisiae* ou un de ses mutants.

En tant que matières premières renouvelables, on peut également utiliser des matériaux comprenant de la cellulose ou de l'hémicellulose voire de la lignine qui, en présence des microorganismes adéquats, peuvent être transformées en matières comprenant du sucre. Parmi ces matières renouvelables, on compte la paille, le bois, le papier. Ces matériaux peuvent avantageusement provenir de matériaux récupérés.

Les listes présentées ci-dessus ne sont pas limitatives.

De préférence, l'étape de fermentation est suivie d'une étape de purification destinée à séparer l'éthanol des autres alcools.

A l'étape b) est mise en oeuvre la déshydratation du ou des alcools obtenus pour produire, dans un premier réacteur, au moins un alcène choisi parmi l'éthylène et les mélanges d'alcènes comprenant de l'éthylène, le produit secondaire de la déshydratation étant de l'eau.

Généralement, la déshydratation de l'alcool est effectuée à l'aide d'un catalyseur à base d'alpha-alumine comme le catalyseur commercialisé par EUROSUPPORT sous la dénomination commerciale ESM 110 ® (alumine trilobique non dopée contenant peu -environ 0,04%- de Na₂O résiduel).

Les conditions opératoires de la déshydratation font partie des connaissances générales de l'homme du métier, à titre indicatif, la déshydratation est généralement effectuée à une température de l'ordre de 400 °C.

Un autre avantage du procédé selon l'invention est son économie en énergie : les étapes de fermentation et déshydratation du procédé selon l'invention sont effectuées à des températures relativement basses, inférieures à 500°C, de préférence inférieures à 400°C, en comparaison l'étape de craquage et de vapocraquage du pétrole en éthylène s'effectue à une température de l'ordre de 800°C.

Cette économie d'énergie s'accompagne aussi d'une diminution du taux de CO₂ émis dans l'atmosphère.

De préférence une étape de purification est effectuée lors de l'étape a) ou lors de l'étape b).

Les étapes éventuelles de purification (purification de(s) alcool(s) obtenus à l'étape a), purification de(s) alcène(s) obtenus à l'étape b)) sont avantageusement conduites par absorption sur des filtres classiques tels que tamis moléculaires, zéolithes, noir de carbone....

Si l'alcool obtenu à l'étape a) a été purifié de façon à isoler l'éthanol, l'alcène obtenu à l'étape b) est de l'éthylène.

Si l'alcool obtenu à l'étape a) n'a pas été purifié, on obtient à l'issue de l'étape b) un mélange d'alcènes comprenant de l'éthylène.

Avantageusement, on effectue au moins une étape de purification lors de l'étape a) et/ou de l'étape b) afin d'obtenir de l'éthylène de degré de pureté suffisant pour effectuer une copolymérisation.

De manière particulièrement préférée, l'alcool obtenu à l'étape a) est purifié de façon à isoler l'éthanol, en conséquence l'alcène obtenu à l'étape b) est l'éthylène.

Les principales impuretés présentes dans l'éthylène issu de la déshydratation de l'éthanol sont l'éthanol, le propane et l'acétaldéhyde.

Avantageusement, l'éthylène devra être purifié, c'est-à-dire que l'éthanol, le propane et l'acétaldéhyde devront être éliminés, pour pouvoir copolymériser facilement à l'étape c).

L'éthylène, l'éthanol, le propane et l'acétaldéhyde peuvent être séparés en mettant en oeuvre une ou plusieurs distillations à basse température.

Les températures d'ébullition de ces composés sont les suivantes :

| composé | température d'ébullition (°C) |
|---|---|
| éthylène | -103,7 |
| propane | -42,1 |
| acétaldéhyde | 20,8 |
| éthanol | 75,5 |

L'éthylène, l'éthanol, le propane et l'acétaldéhyde sont refroidis à environ -105°C, de préférence -103,7°C puis distillés pour extraire l'éthylène.

Un autre avantage du procédé selon la présente invention concerne les impuretés. Les impuretés présentes dans l'éthylène issu de la déshydratation de l'éthanol sont totalement différentes de celles présentes dans l'éthylène issu de vapocraquage. En particulier parmi les impuretés présentes dans l'éthylène issu de vapocraquage, on compte le dihydrogène et le méthane et ceci quelle que soit la composition de la charge initiale.

Classiquement la séparation du dihydrogène et du méthane s'effectue après compression à 36 bar et refroidissement à environ -120°C. Dans ces conditions le dihydrogène et le méthane, liquides, sont séparés dans le déméthaniseur ; puis l'éthylène est récupéré à 19 bar et -33°C.

Le procédé selon la présente demande permet de s'affranchir de l'étape de séparation du dihydrogène et du méthane, et permet également de refroidir le mélange à -105°C à pression atmosphérique au lieu de -120°C à 36 bar. Le refroidissement de cette étape de séparation peut se faire aussi sous pression pour augmenter la température d'ébullition des composés à séparer (par exemple vers 20 bar et -35°C). Ces différences contribuent également à rendre le procédé selon l'invention plus économique (économie de matériel et économie d'énergie qui s'accompagne aussi d'une diminution du taux de CO₂ émis dans l'atmosphère).

Un autre avantage est que l'éthylène obtenu à l'étape b) du procédé selon l'invention ne comprend pas d'acétylène contrairement à l'éthylène obtenu par craquage ou vapocraquage. Or l'acétylène est très réactif et provoque des réactions secondaires d'oligomérisation, l'obtention d'éthylène sans acétylène est donc particulièrement avantageuse.

Un autre avantage est que le procédé selon l'invention peut être mis en oeuvre dans des unités de production localisées sur le lieu de production des matières premières. En outre, la taille des unités de production du procédé selon l'invention est beaucoup plus petite que la taille d'une raffinerie : les raffineries sont en effet de grosses installations situées généralement loin des centres de production des matières premières et alimentées par des pipelines.

A l'étape c) la copolymérisation des monomères comprenant l'éthylène, l'ester vinylique et éventuellement un autre comonomère, et un initiateur de polymérisation est effectuée par polymérisation en émuision aqueuse ou par polymérisation à haute pression en réacteur autoclave ou tubulaire.

La copolymérisation radicalaire haute pression est généralement effectuée en introduisant l'éthylène, les comonomères (esters vinyliques d'acide carboxylique) et un initiateur de polymérisation à pression élevée dans un réacteur autoclave ou tubulaire à une température comprise entre 80°C et 325°C en réacteur tubulaire et 150 à 290°C en réacteur autoclave. La quantité des comonomères introduits peut aller jusqu'à 60 % en poids par rapport à la quantité totale des monomères (éthylène et comonomère) introduits dans le réacteur, ce qui permet l'obtention d'un copolymère comprenant jusqu'à 60% en poids de comonomère par exemple des esters vinyliques d'acide carboxylique.

La pression est régulée à l'aide d'une vanne de détente située à la sortie du réacteur. On récupère le polymère formé à la sortie du réacteur et le monomère n'ayant pas réagi est préférentiellement recyclé en début de réacteur. La pression à l'intérieur du réacteur est avantageusement comprise entre 500 et 3000 bar, préférentiellement entre 1000 et 2500 bar.

Avec les comonomères et l'initiateur, on peut également utiliser un agent de transfert, cet agent de transfert peut par exemple être un ou plusieurs alcanes tels que butane, pentane, un ou plusieurs alcènes tels que propylène, butène, un ou plusieurs aldéhydes tels que propionaldéhyde, acétaldéhyde ou une ou plusieurs cétones telles que acétone, méthyle éthyle cétone. En ajoutant cet agent de transfert on peut limiter la masse molaire du polymère fabriqué.

A titre d'initiateur de polymérisation, tous les composés organiques et inorganiques qui libèrent des radicaux libres dans les conditions de la réaction pourront être utilisés, de préférence on utilisera des composés ou des mélanges de composés comprenant un groupement peroxyde par exemple les composés suivants tertiobutylperoxyneodecanoate, tertiobutylperoxypivalate, tertioamylperoxypivalate, di (3,5,5 trimethylhexanoyl) peroxyde, didecanoyl peroxyde, tertioamyl peroxy-2-ethylhexanoate, tertiobutyl peroxy-2-ethylhexanoate, tertiobutyl peroxy-3,5,5-trimethylhexanoate, tertioamyl peroxy-3,5,5-trimethylhexanoate, tertiobutyl peroxybenzoate, tertiobutylperoxyacetate ou ditertioamyl peroxyde.

Généralement, la quantité massique d'initiateur de polymérisation est comprise entre 1 et 1000 ppm par rapport à la quantité totale du mélange introduit.

Lorsqu'un réacteur tubulaire est utilisé, l'introduction du mélange de l'éthylène et des comonomères est effectuée de préférence en tête du réacteur tubulaire. L'initiateur ou le mélange d'initiateurs est injecté à l'aide d'une pompe haute pression en tête du réacteur, après l'endroit d'introduction du mélange de l'éthylène et des comonomères.

Le mélange de l'éthylène et des comonomères peut être injecté en au moins un autre point du réacteur, cette injection est elle-même suivie d'une nouvelle injection d'initiateur ou d'un mélange d'initiateurs, on parle alors de technique d'injection multipoint. Lorsque la technique d'injection multipoint est utilisée, le mélange est préférentiellement injecté de manière telle que le rapport pondéral du mélange injecté en entrée de réacteur sur la totalité du mélange injecté est compris entre 10 et 90%.

D'autres procédés de copolymérisation haute pression tubulaire utilisables sont par exemple ceux décrits dans US2006/0149004 A1 ou dans US2007/0032614 A1.

On peut également utiliser un réacteur autoclave pour réaliser la polymérisation haute pression radicalaire.

Un réacteur autoclave consiste généralement en un réacteur cylindrique dans lesquels est placé un agitateur. Le réacteur peut être séparé en plusieurs zones reliées entre elles en série. Avantageusement, le temps de séjour dans le réacteur est compris entre 30 et 120 secondes. Préférentiellement le rapport longueur/diamètre du réacteur est compris entre 3 et 25. L'éthylène seul et le ou les comonomères sont injectés dans la première zone du réacteur à une température comprise entre 20 et 120°C, préférentiellement entre 50 et 80°C. Un initiateur est également injecté dans cette première zone réactionnelle. Si le réacteur est un réacteur multizones, le flux d'éthylène et de comonomères n'ayant pas réagi ainsi que le polymère formé passent alors dans les zones réactionnelles suivantes. Dans chaque zone réactionnelle, de l'éthylène, des comonomères et des initiateurs peuvent être injectés. La température des zones est comprise entre 150°C et 290°C et préférentiellement entre 160°C et 280°C. La pression du réacteur est comprise entre 500 et 3000 bar préférentiellement entre 1200 et 2200 bar.

D'autres procédés de copolymérisation utilisables sont par exemples ceux décrits dans les demandes de brevets FR2660660, FR2498609, FR2569411 et FR2569412.

La polymérisation en émulsion permet la fabrication de copolymères comprenant un taux massique de comonomères compris entre 40 et 99%. Ces copolymères peuvent être copolymérisés à basse pression, c'est-à-dire une pression inférieure à 50 bar, les monomères étant en émulsion dans l'eau. On peut utiliser par exemple les procédés décrits dans les brevets US 7189461, US 5143966 ou US 6319978.

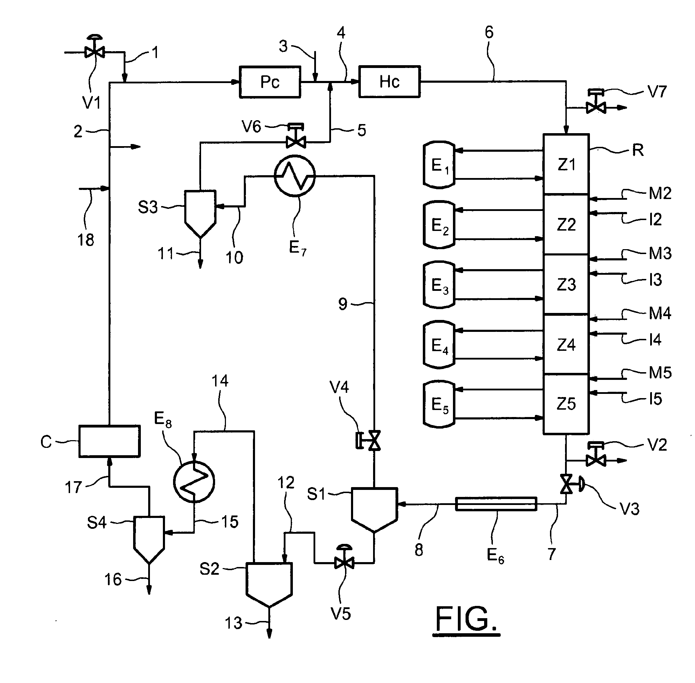

Sur la figure unique jointe en annexe est présenté un dispositif permettant la mise en oeuvre du procédé de copolymérisation selon l'invention suivant une technique d'injection des réactifs en plusieurs points.

Ce dispositif comprend un réacteur tubulaire R comprenant cinq zones Z1, Z2, Z3, Z4 et Z5, un séparateur moyenne pression S1 qui constitue l'entrée dans un circuit de recyclage moyenne pression et un séparateur basse pression S2 qui constitue l'entrée dans un circuit de recyclage basse pression.

Le circuit de recyclage moyenne pression comprend le séparateur moyenne pression S1, la conduite 9 munie de la vanne V4, l'échangeur de chaleur E7, la conduite 10, le séparateur S3 et la conduite 5, munie de la vanne V6.

Le circuit de recyclage basse pression comprend le séparateur basse pression S2, la conduite 14, l'échangeur de chaleur E8, la conduite 15, le séparateur S4, la conduite 17, le compresseur C, la conduite 2.

Le réacteur tubulaire R est un tube à double enveloppe dans laquelle circule de l'eau destinée à apporter ou retirer des calories en vue de chauffer ou de refroidir le fluide circulant dans le réacteur. Le réacteur tubulaire R comprend cinq zones Z1, Z2, Z3, Z4 et Z5 auxquelles correspondent cinq parties de la double enveloppe : E1 est la partie de la double enveloppe située autour de la zone Z1, E2 est la partie de la double enveloppe située autour de la zone Z2, E3 est la partie de la double enveloppe située autour de la zone Z3, E4 est la partie de la double enveloppe située autour de la zone Z4, E5 est la partie de la double enveloppe située autour de la zone Z5 ; le débit et la température de l'eau circulant dans chacune des parties E1, E2, E3 , E4 et E5 peuvent être différents.

Selon ce dispositif, de l'éthylène frais circulant dans la conduite 1 (à une pression de 60 bar) est admis dans la conduite 2 du dispositif. La conduite 1 est munie d'une vanne de détente V1.

Dans la conduite 2, cet éthylène frais est mélangé à un flux gazeux (recyclage de l'éthylène et du ou des comonomères du circuit de recyclage basse pression) provenant du compresseur C.

La conduite 2 amène le mélange dans un précompresseur Pc (où le mélange est comprimé de 60 bar à 200 bar) puis le mélange sort du précompresseur Pc par la conduite 4. Des comonomères frais sont introduits dans la conduite 4 au moyen de la conduite 3. En aval de la conduite 3, la conduite 5 introduit dans la conduite 4 le mélange de fluides recyclés provenant du circuit de recyclage moyenne pression.

Le mélange circulant dans la conduite 4 est introduit dans l'hypercompresseur Hc (où le mélange est comprimé de 200 bar à une pression comprise entre 1200 et 2500 bar qui est la pression dans le réacteur) puis le mélange sort de l'hypercompresseur Pc par la conduite 6.

La pression à l'intérieur du réacteur est régulée par la vanne de détente V2.

Au besoin, la vanne V7 permet de réguler la pression du mélange dans la conduite 6.

Le réacteur utilisé comprend 5 zones : le mélange comprenant l'éthylène et le ou les comonomères et un agent de transfert est admis dans la zone Z1 du réacteur (en entrée de réacteur) au moyen de la conduite 6.

Dans la zone Z1, le mélange est chauffé jusqu'à la température d'initiation de la réaction de polymérisation (entre 90 et 170°C).

En entrée de la zone Z2, un mélange d'éthylène et de comonomères peut être introduit au moyen de le conduite M2 et un initiateur de polymérisation (généralement au moins un péroxyde et/ou de l'oxygène moléculaire) peut être introduit au moyen de la conduite I2.

Puis dans les zones Z3, Z4 et Z5, les conduites M3, M4 et M5 respectivement permettent d'introduire des ajouts de mélanges d'éthylène et de comonomères et les conduites 13, I4 et I5 respectivement permettent d'introduire des ajouts d'initiateur de polymérisation.

La réaction de polymérisation est fortement exothermique, la température du mélange qui passe dans le réacteur tubulaire augmente progressivement.

Une partie des calories générées par la réaction de copolymérisation dans les zones Z1, Z2, Z3, Z4 et Z5 est récupérée par l'eau circulant dans la partie de la double enveloppe correspondante respectivement E1, E2, E3, E4 et E5.

Quand on effectue des ajouts au moyen des conduites M2 à M5 et 12 à 15, dans chaque zone Z1, Z2, Z3, Z4 et Z5, on obtient un profil de température identique avec une montée de la température jusqu'à un pic compris entre 180°C et 325°C (le début de la montée en température étant dû à l'injection de l'initiateur de polymérisation et du mélange) puis une diminution de la température (qui correspond à la fin de la réaction de polymérisation et au refroidissement du flux par la double enveloppe).

Avant la sortie du réacteur, le mélange comprenant le polymère est refroidi à une température comprise entre 140°C et 240°C.

Le mélange comprenant le polymère sort du réacteur par la conduite 7 munie d'une vanne V3 qui permet de détendre le mélange à environ 260 Bar. Puis le mélange entre dans l'échangeur de chaleur E6 où il est refroidi et en sort par la conduite 8.

La conduite 8 amène le mélange dans le séparateur moyenne pression S1. Dans S1 le copolymère formé est séparé du mélange des produits qui n'ont pas réagi : éthylène, comonomères et agent de transfert.

Le mélange des produits qui n'ont pas réagi est amené dans l'échangeur de chaleur E7 par la conduite 9 où il est refroidi puis sort de l'échangeur de chaleur E7 par la conduite 10. La conduite 10 amène les produits dans le séparateur S3 où les cires de polymères (possédant une masse basse et qui n'ont pas été séparées dans le séparateur SI) sont isolées et extraites du dispositif par la conduite 11. La conduite 5, munie de la vanne V6, amène le mélange éthylène, comonomères et agent de transfert du séparateur S3 dans la conduite 4.

Le copolymère sort du séparateur S1 par la conduite 12 munie d'une vanne V5 et est introduit dans le séparateur basse pression S2. La vanne V5 permet de détendre le copolymère à une pression d'environ 2 à 5 bars. Le copolymère est extrait du dispositif par la conduite 13 puis il est convoyé vers une extrudeuse pour être transformé en granulés.

La conduite 14 permet d'évacuer le mélange de gaz (éthylène/comonomères, agent de transfert qui n'ont pas été séparées dans le séparateur S1), ce mélange est amené dans l'échangeur de chaleur E8 par la conduite 14 où il est refroidi jusqu'à environ 35°C puis sort de l'échangeur de chaleur E8 par la conduite 15. La conduite 15 amène les produits dans le séparateur S4 où les monomères sont condensés. Une partie des monomères est extraite du dispositif par la conduite 16, l'autre partie des monomères est introduite par la conduite 17 dans le compresseur C (où ils sont comprimés à 60 bar).

Les monomères sortent du compresseur par la conduite 2. La conduite 18 permet d'introduire l'agent de transfert dans la conduite 2.

A l'étape d), le copolymère obtenu est isolé et éventuellement purifié, selon une technique classique, en fonction de l'application auquel il est destiné.

Selon une variante particulière, on pourra utiliser de l'ester vinylique, et notamment de l'acétate de vinyle et du propionate de vinyle comprenant des atomes de carbone d'origine renouvelable.

Ces esters vinyliques peuvent être obtenus selon les procédés décrits dans la demande FR0854976 de la Demanderesse.

L'acétate de vinyle est alors susceptible d'être obtenu selon le procédé comprenant les étapes suivantes :
a) fermentation de matières premières renouvelables et, éventuellement purification pour produire au moins un alcool choisi parmi l'éthanol et les mélanges d'alcools comprenant de l'éthanol ;
b) séparation de l'alcool obtenu en deux parties, puis introduction de la première partie dans un premier réacteur et de la deuxième partie dans un deuxième réacteur,
c) dans le premier réacteur, déshydratation de l'alcool obtenu pour produire au moins un alcène choisi parmi l'éthylène et les mélanges d'alcènes comprenant de l'éthylène et, éventuellement purification de l'éthylène ;
d) dans le deuxième réacteur, oxydation de l'alcool obtenu pour produire de l'acide acétique et, éventuellement purification de l'acide acétique;
e) introduction dans un troisième réacteur de l'éthylène obtenu à l'issue de l'étape c) et de l'acide acétique obtenu à l'issus de l'étape d), et mise en oeuvre de la réaction d'acyloxylation de l'éthylène,
f) isolation et éventuellement purification de l'acétate de vinyle obtenu à l'issue de l'étape e).

Et le propionate de vinyle est susceptible d'être obtenu selon le procédé comprenant les étapes suivantes :
a. fermentation de matières premières renouvelables et, éventuellement purification pour produire au moins un alcool choisi parmi l'éthanol et les mélanges d'alcools comprenant de l'éthanol ;
b. déshydratation de l'alcool obtenu pour produire, dans un premier réacteur, au moins un alcène choisi parmi l'éthylène et les mélanges d'alcènes comprenant de l'éthylène et, éventuellement purification de l'éthylène ;
c. déshydratation de glycérol dans un deuxième réacteur, en présence d'oxygène moléculaire pour produire de l'acide acrylique,
d. hydrogénation de l'acide acrylique en présence d'oxygène moléculaire pour produire de l'acide propanoïque,
e. introduction dans un troisième réacteur de l'éthylène obtenu à l'issue de l'étape b) et de l'acide propanoïque obtenu à l'issus de l'étape d), et mise en oeuvre de la réaction de acyloxylation de l'éthylène,
f. isolation et éventuellement purification du propionate de vinyle obtenu à l'issue de l'étape e).

L'éthylène utilisé à l'étape c) du procédé de fabrication d'acétate de vinyle et utilisé à l'étape b) du procédé de fabrication de propionate de vinyle est alors obtenu de la même manière avantageuse que l'éthylène utilisé dans les comonomères selon la présente invention.

Le procédé de fabrication des terpolymères d'éthylène, d'au moins un ester vinylique et d'au moins un anhydride d'acide carboxylique insaturé selon la présente demande met en oeuvre à l'étape c) une réaction de copolymérisation de l'éthylène avec au moins un ester vinylique avantageusement l'acétate de vinyle ou le propionate de vinyle, et au moins un anhydride d'acide carboxylique insaturé avantageusement l'anhydride maléique. Cette étape est conduite de la même manière que l'étape c) de fabrication des copolymères d'éthylène et d'au moins un ester vinylique.

Selon une variante particulière, on pourra utiliser un ester vinylique, et/ou de l'anhydride maléique comprenant des atomes de carbone d'origine renouvelable.

Ces esters vinyliques peuvent être obtenus selon les procédés mentionnés ci-dessus et décrits dans la demande FR0854976 de la Demanderesse.

L'anhydride maléique peut être obtenu selon le procédé décrit dans la demande FR 0854896 de la Demanderesse, comprenant les étapes suivantes :
a) fermentation de matières premières renouvelables et, éventuellement purification pour produire un mélange comprenant au moins du butanol ;
b) oxydation du butanol en anhydride maléique à une température généralement comprise entre 300 et 600 °C, au moyen d'un catalyseur à base d'oxydes de vanadium et/ou de molybdène ;
   isolation de l'anhydride maléique obtenu à l'issus de l'étape b).

De préférence, les copolymère obtenus selon l'invention sont choisis parmi :
■ les copolymères statistiques d'éthylène et d'au moins un ester vinylique en particulier les copolymères statistiques d'éthylène et d'acétate de vinyle et les copolymères statistiques d'éthylène et de propionate de vinyle ;
■ les terpolymères statistiques d'éthylène, d'au moins un ester vinylique et d'anhydride maléique, en particulier les terpolymères statistiques d'éthylène, d'acétate de vinyle et d'anhydride maléique et les copolymères statistiques d'éthylène, de propionate de vinyle et d'anhydride maléique ;
■ les terpolymères d'éthylène, d'acétate de vinyle et d'un ester (meth)acrylique d'acide carboxylique ;
■ les copolymères statistiques d'éthylène et d'au moins un ester vinylique greffés par un anhydride d'acide carboxylique insaturé en particulier les copolymères statistiques d'éthylène et d'acétate de vinyle greffés par l'anhydride maléique.

Avantageusement les esters d'acide carboxylique insaturés utilisés dans les terpolymères obtenus selon l'invention sont des (méth)acrylates d'alkyle, le nombre d'atomes de carbone de la partie alkyle des (méth)acrylates d'alkyle va de préférence de 1 à 24, de manière particulière les (méth)acrylates d'alkyle sont choisis parmi l'acrylate de méthyle, l'acrylate d'éthyle, l'acrylate de n-butyle, l'acrylate d'isobutyle, l'acrylate de 2-éthylhexyle. De particulièrement préférée, on utilise l'acrylate de méthyle, l'acrylate de n-butyle et l'acrylate de 2-éthylhexyle.

Les copolymères obtenus selon l'invention comprennent préférentiellement de 40 à 99% d'éthylène en masse et de 1 à 60% d'ester vinylique d'acide carboxylique ; de manière plus préférée de 55 à 90% d'éthylène en masse et de 10 à 45% d'ester vinylique d'acide carboxylique).

Les terpolymères obtenus selon l'invention comprennent préférentiellement de 40 à 99% d'éthylène en masse, de 0,99 à 50% d'ester vinylique d'acide carboxylique en masse et 0,01 à 10% d'anhydride maléique ou d'ester (méth)acrylique ; de manière plus préférée de 60% à 94,95% d'éthylène en masse, de 5 à 35% d'ester vinylique d'acide carboxylique en masse et 0,05 à 5% d'anhydride maléique ou d'ester (méth)acrylique.

L'indice de fluidité à l'état fondu MFI (en anglais Melt Fluid Index) de ces copolymères est avantageusement compris dans la gamme allant de 0,1 à 1000 g/10min (ASTM D 1238, 190°C, 2,16kg), préférentiellement de 1 à 500 g/10min.

Le copolymère ou le terpolymère peut être amorphe ou semi-cristallin. Lorsqu'il est semi-cristallin, sa température de fusion peut être comprise entre 45°C et 115°C.

Selon un mode particulier de l'invention, le copolymère est partiellement ou totalement saponifié, c'est à dire que la fonction ester de vinyle du copolymère est hydrolysée pour former une fonction alcool. On peut effectuer cette saponification par les techniques connues de l'homme du métier. En réalisant la saponification du copolymère éthylène- acétate de vinyle (EVA), on obtient un copolymère éthylène-acétate de vinyle saponifié (EVOH), c'est à dire qu'au moins une partie des fonctions acétate de vinyle du copolymère réagissent pour former de l'alcool de vinyle.

Ces copolymères saponifiés ont d'excellentes propriétés barrières aux gaz leur permettant d'être avantageusement utilisés dans les structures multicouches, en particulier dans l'emballage alimentaire.

La demande decrit également sur des compositions comprenant, en plus du copolymère ou du terpolymère, au moins un additif pour améliorer les propriétés du matériau final.

Parmi ces additifs, on compte les antioxydants ; les agents de protection UV ; agents dits « de mise en oeuvre » ayant pour fonction d'améliorer l'aspect du polymère final lors de sa mise en oeuvre telles que les amides grasses, l'acide stéarique et ses sels, l'éthylène bis-stéaramide ou les polymères fluorés ; les agents anti-buée ; les agents anti-bloquants tels que la silice ou le talc ; les charges telles que le carbonate de calcium et les nanocharges comme par exemple les argiles ; les agent de couplage tels les silanes ; les agents réticulants comme les peroxydes ; les agents antistatiques ; les agents nucléants ; les pigments ; les colorants. Ces additifs sont généralement utilisés dans des teneurs comprises entre 10 ppm et 100 000 ppm en poids par rapport au poids du copolymère final. Les compositions peuvent comprendre également des additifs choisis parmi les plastifiants, les fluidifiants, les additifs retardateurs de flamme, tels les hydroxydes d'aluminium ou de magnésium (ces derniers additifs peuvent atteindre des quantités bien supérieures à 100000 ppm). Certains de ces additifs peuvent être introduits dans la composition sous forme de mélanges-maîtres.Ces compositions, peuvent également comprendre d'autres polymères, tels que les polyoléfines différentes des copolymères selon l'invention, le polyamide ou le polyester.

A titre d'exemples de polyoléfines différentes du polymère obtenu selon l'invention, on peut citer les homopolymères et copolymères de l'éthylène comme le polyéthylène très basse densité (PETBD), le polyéthylène basse densité (PEBD), le polyéthylène basse densité linéaire (PEBDL), le polyéthylène moyenne densité (PEMD), le polyéthylène haute densité (PEHD), les copolymères comprenant de l'éthylène et de l'acétate de vinyle ou des copolymères comprenant de l'éthylène et du (méth)acrylate d'alkyle dont l'éthylène n'est pas issu de matières premières renouvelables.

A partir des copolymères, on peut également former des films, par exemple des films encapsulant pour les panneaux solaires, les films agricoles, les films d'emballage, les films thermo-adhésifs, les films de protection.

La présente demande vise encore les utilisations des copolymères obtenus selon l'invention et les compositions comprenant au moins un copolymère obtenu selon l'invention, notamment les utilisations des copolymères selon l'invention en tant qu'adhésifs ou que compositions adhésives dans une structure multicouche.

La présente demande vise en particulier les utilisations des copolymères et des compositions obtenus selon l'invention en tant qu'adhésifs ou compositions adhésives notamment, en coextrusion, en extrusion couchage ou en extrusion lamination. Ces copolymères obtenus selon l'invention présentent une adhésion à de nombreux supports tels que les métaux ou les polymères comme les polyesters, les polyamides, les polyoléfines, ou les polymères qui présentent des propriétés barrière à l'eau, aux gaz ou aux hydrocarbures.

En coextrusion, on peut citer parmi les supports coextrudables tous types de polymères tels que le polyéthylène téréphtalate (PET), le polypropylène (PP), le polychlorure de vinyle (PVC), le chlorure de polyvinylidène (PVDC), le polyfluorure de vinyle (PVF), le fluorure de polyvinylidène (PVDF), le polyamide (PA), le polystyrène (PS), ... Les adhésifs et compositions adhésives peuvent ainsi être utilisés dans des structures multicouches, notamment entre une couche de polyéthylène téréphtalate (PET) et une couche de polyéthylène ou entre une couche de polyester issu de matières renouvelables comme par exemple le poly(acide lactique) et un polymère ayant des propriétés barrière, comme par exemple le copolymère éthylène-acétate de vinyle saponifié (EVOH) ou le PA qui ont des propriétés barrière à l'oxygène.

Ainsi la demande concerne une structure multicouche obtenue par utilisation de la composition adhésive dans un procédé d'extrusion-couchage pour application sur un support, ledit support étant choisi parmi l'aluminium, le papier ou carton, la cellophane, les films à base de résines polyéthylène, polypropylène, polyamide, polyester, chlorure de polyvinyle (PVC), chlorure de polyvinylidène (PVDC), polyacrylonitryle (PAN), ces films étant orientés ou non, métallisés ou non, traités ou non par voie physique ou chimique, et les films revêtus d'une fine couche barrière inorganique tels que le polyester (PET SiOx ou AlOx).

La demande concerne aussi une structure multicouche obtenue par utilisation de la composition adhésive, dans un procédé d'extrusion-lamination pour coller entre eux plusieurs supports de nature différente, les supports sont généralement choisis parmi l'aluminium, le papier ou carton, la cellophane, les films à base de résines polyéthylène, polypropylène, polyamide, polyester, chlorure de polyvinyle (PVC), chlorure de polyvinylidène (PVDC), polyacrylonitryle (PAN), ces films étant orientés ou non, métallisés ou non, traités ou non par voie physique ou chimique, et les films revêtus d'une fine couche barrière inorganique, tels que le polyester (PET SiOx ou AlOx).

La présente demande vise également l'utilisation les compositions en tant que couche de scellage notamment sur un matériau choisi parmi l'aluminium, les polystyrènes (PS), les polypropylènes (PP), les polyamines (PA)...

Les copolymères obtenus selon l'invention peuvent aussi être utilisés :
■ en tant que compatibilisant de composés (c'est-à-dire un adjuvant qui permet d'améliorer la compatibilité avec lesdits composés), notamment les fibres naturelles, les alliages polyoléfines/polyamine (PO/PA), les alliages polyoléfines/polyester ou biopolyester, les alliages polyoléfines/amidons...
■ en tant que modifiant choc dans des polymères (c'est-à-dire en tant qu'adjuvant dans un polymère permettant d'améliorer la résistance aux chocs dudit polymère) tels que les polymères polyéthylène téréphtalate (PET), polyamide (PA), les polypropylènes (PP), les polybutylène téréphtalate (PBT), les biopolyesters (acide polylactique (PLA), polyhydroxyalkanoates (PHA)...)

A partir des copolymères, on peut également former des films, tels que des films encapsulant pour les panneaux solaires. Une composition préférée pour la fabrication d'un film encapsulant comprend un mélange d'un copolymère statistique d'éthylène et d'acétate de vinyle avec un terpolymère statistique d'éthylène, d'acétate de vinyle et d'anhydride maléique, ce copolymère et/ou ce terpolymère étant selon l'invention. On peut aussi former des revêtements souples notamment pour la construction pour le sol ou les murs ou dans l'automobile, des films agricoles, des films thermo-adhésifs, des films de protection ou des films d'emballage.

On peut utiliser les copolymères et compositions obtenus selon l'invention comme additifs dans le pétrole ou les carburants. Ces copolymères peuvent aussi entrer dans la composition d'une encre. Dans ces applications, les copolymères éthylène-ester vinylique-ester (méth)acrylique d'acide carboxylique sont particulièrement avantageux.

Ces copolymères peuvent aussi être utilisés pour fabriquer une masse insonorisante c'est-à-dire une masse expansible réticulable ayant une fonction insonorisante. Des pièces souples peuvent également être formées à partir des copolymères de l'invention par injection ou thermoformage ; on peut également fabriquer des tubes ou des récipients tels que des bouteilles ou des réservoirs par extrusion de tube ou par extrusion « blow molding ».

Les copolymères obtenus selon l'invention peuvent également être contenus dans des compositions pour fabriquer des textiles tissés ou non-tissés.

Une autre application possible pour les copolymères obtenus selon l'invention est de fabriquer des mélanges-maîtres. On peut également utiliser les copolymères selon l'invention pour fabriquer des câbles électriques. En particulier, on peut les utiliser pour fabriquer une gaine de câble électrique. On peut également fabriquer des compositions en dispersant un composé conducteur (par exemple du noir de carbone) pour former des compositions mi-conductrices mi-isolantes (couramment appelées semi-conductrices) ; ces compositions sont particulièrement utiles pour fabriquer des câbles moyenne ou haute tension.

Ces copolymères peuvent être également utilisés en tant que modifiant de bitume. Le copolymère obtenus selon l'invention peut également rentrer dans la composition d'un adhésif thermo-fusible (communément appelé Hot-melt).

En particulier, on peut formuler une composition d'adhésif thermofusible en mélangeant aux copolymères obtenus selon l'invention des résines dites « tackifiantes », des cires et des antioxydants. On peut également y ajouter d'autres additifs tels que les plastifiants, les fluidifiants, les pigments ou les charges.

Les résines dites « tackifiantes » peuvent être solides ou liquides, utilisées seules ou en mélange ; elles permettent principalement d'apporter du pouvoir adhésif à la composition. On peut citer parmi celles-ci:
■ les résines à base d'ester de colophane naturelle ou modifiée, par exemple polymérisée, notamment les esters de pentaérythritol ou de gycérol, les résines terpéniques ou polyterpéniques modifiées,
■ les résines synthétiques de type alpha-méthyl-styrène, styrène/terpène, terpène/phénol,
■ les résines d'origines hydrocarbures telles que les résines aliphatiques ou aromatiques, non hydrogénées ou hydrogénées totalement ou partiellement,
■ les cires, qui permettent de régler la fluidité et le temps de prise de l'adhésif, peuvent être choisies parmi les familles suivantes : les paraffines, les cires microcristallines, les cires de polyéthylène, les cires Fischer-Tropsch oxydées ou non, les cires fonctionnalisées type hydroxystéaramide ou amides grasses.

### EXEMPLE

Un copolymère d'éthylène et d'acétate de vinyle selon la présente invention a été préparé à partir d'éthylène obtenu en mettant en oeuvre les étapes a) et b) selon le procédé de la présente demande, puis en effectuant un copolymérisation (étape c)) au moyen du dispositif décrit ci-dessus et présenté sur la figure unique jointe en annexe. Le réacteur tubulaire utilisé mesure 600 m de long et 42 mm de diamètre. L'éthylène a été injecté au débit de 12 tonnes/heure (conduite 1), et 800 kg/heure d'acétate de vinyle (conduite 3), le mélange est comprimé dans l'hypercompresseur (Hc) à 2400 bar. Le mélange est préchauffé à 120°C dans la zone Z1, puis un mélange Lup 11/26 (c'est-à-dire Luperox 11 Tert-butylperoxypivalate /Luperox 26 Tert-butylperoxy-2-éthylhexanoate) est injecté par la conduite (12). Dans la zone Z2, la température monte jusqu'à 210°C puis redescend à 160°C en sortie de zone Z2. Puis dans chaque zone Z3, Z4 et Z5, à l'entrée de la zone on réinjecte un mélange Lup 11/26, la température monte jusqu'à 210°C puis redescend à 160°C en sortie de zone. On obtient 1,7 tonnes/heure de copolymères éthylène/acétate de vinyle avec une teneur en acétate de vinyle de 6% poids et un Melt Index de 0,5.

## Revendications

1. Procédé de fabrication d'un copolymère de l'éthylène et d'au moins un ester vinylique d'acide carboxylique comprenant les étapes suivantes :
a) fermentation de matières premières renouvelables et, éventuellement purification pour produire au moins un alcool choisi parmi l'éthanol et les mélanges d'alcools comprenant de l'éthanol ;
b) déshydratation de l'alcool obtenu pour produire au moins un alcène choisi parmi l'éthylène et les mélanges d'alcènes comprenant de l'éthylène et, éventuellement purification de l'alcène pour obtenir de l'éthylène,
c) copolymérisation de l'éthylène avec au moins un comonomère choisi parmi les esters vinyliques d'acides carboxyliques,
d) isolation du copolymère obtenu.

2. Procédé de fabrication d'un terpolymère de polyéthylène, d'au moins un ester vinylique et d'anhydride d'acide carboxylique insaturé comprenant les étapes suivantes :
a) fermentation de matières premières renouvelables et, éventuellement purification pour produire au moins un alcool choisi parmi l'éthanol et les mélanges d'alcools comprenant de l'éthanol ;
b) déshydratation de l'alcool obtenu pour produire au moins un alcène choisi parmi l'éthylène et les mélanges d'alcènes comprenant de l'éthylène et, éventuellement purification de l'alcène pour obtenir de l'éthylène,
c) copolymérisation de l'éthylène avec au moins un ester vinylique et au moins un anhydride d'acide carboxylique insaturé,
d) isolation du copolymère obtenu.

3. Procédé de fabrication d'un copolymère selon la revendication 1 ou 2, **caractérisé en ce que** les matières premières renouvelables sont des matières végétales choisies parmi la canne à sucre et la betterave sucrière, l'érable, le palmier-dattier, le palmier à sucre, le sorgho, l'agave américain, le maïs, le blé, l'orge, le sorgho, le froment, le riz, la pomme de terre, le manioc, la patate douce, les algues, les matériaux comprenant de la cellulose ou de l'hémicellulose tels que le bois, la paille ou le papier.

4. Procédé de fabrication d'un copolymère selon l'une quelconque des revendications 1 à 3 **caractérisé en ce que** la copolymérisation de l'étape c) est une copolymérisation radicalaire haute pression effectuée en introduisant l'éthylène, le ou les comonomères et un initiateur de polymérisation à pression élevée soit dans un réacteur tubulaire à une température comprise entre 80 et 325°C, ou dans un réacteur autoclave à une température comprise entre 150 et 290°C, la quantité des comonomères introduite dans le réacteur tubulaire ou autoclave allant jusqu'à 60 % en poids par rapport à la quantité totale éthylène et comonomères introduite dans le réacteur.

5. Procédé de fabrication d'un copolymère selon la revendication 4 **caractérisé en ce que** la copolymérisation de l'étape c) est effectuée dans un réacteur tubulaire, le mélange de l'éthylène et des comonomères étant injecté au moins en un autre point du réacteur, cette injection étant elle-même suivie d'une nouvelle injection d'initiateur ou de mélange d'initiateurs.

## Patentansprüche

1. Verfahren zur Herstellung eines Copolymers von Ethylen und mindestens einem Carbonsäurevinylester mit den folgenden Schritten:
a) Fermentation von nachwachsenden Rohstoffen und gegebenenfalls Reinigung zur Herstellung von mindestens einem Alkohol, der aus Ethanol und ethanolhaltigen Alkoholgemischen ausgewählt ist;
b) Dehydratisierung des erhaltenen Alkohols zur Herstellung von mindestens einem Alken, das aus Ethylen und ethylenhaltigen Alkengemischen ausgewählt ist, und gegebenenfalls Reinigung des Alkens zum Erhalt von Ethylen;
c) Copolymerisation des Ethylens mit mindestens einem aus Carbonsäurevinylestern ausgewählten Comonomer;
d) Isolierung des erhaltenen Copolymers.

2. Verfahren zur Herstellung eines Terpolymers von Ethylen, mindestens einem Vinylester und einem ungesättigten Carbonsäureanhydrid mit den folgenden Schritten:
a) Fermentation von nachwachsenden Rohstoffen und gegebenenfalls Reinigung zur Herstellung von mindestens einem Alkohol, der aus Ethanol und ethanolhaltigen Alkoholgemischen ausgewählt ist;
b) Dehydratisierung des erhaltenen Alkohols zur Herstellung von mindestens einem Alken, das aus Ethylen und ethylenhaltigen Alkengemischen ausgewählt ist, und gegebenenfalls Reinigung des Alkens zum Erhalt von Ethylen;
c) Copolymerisation des Ethylens mit mindestens einem Vinylester und mindestens einem ungesättigten Carbonsäureanhydrid;
d) Isolierung des erhaltenen Copolymers.

3. Verfahren zur Herstellung eines Copolymers nach Anspruch 1 oder 2, **dadurch gekennzeichnet, dass** es sich bei den nachwachsenden Rohstoffen um pflanzliche Stoffe, ausgewählt aus Zuckerrohr und Zuckerrübe, Ahorn, Dattelpalme, Zuckerpalme, Sorghum, amerikanischer Agave, Mais, Weizen, Gerste, Reis, Kartoffel, Maniok, Süßkartoffel, Algen und Stoffen, die Cellulose oder Hemicellulose umfassen, wie Holz, Stroh oder Papier, handelt.

4. Verfahren zur Herstellung eines Copolymers nach einem der Ansprüche 1 bis 3, **dadurch gekennzeichnet, dass** es sich bei der Copolymerisation von Schritt c) um eine radikalische Hochdruckcopolymerisation handelt, die durch Eintragen von Ethylen, dem Comonomer bzw. den Comonomeren und einem Polymerisationsinitiator bei hohem Druck entweder in einen Rohrreaktor bei einer Temperatur zwischen 80 und 325°C oder in einen Autoklavenreaktor bei einer Temperatur zwischen 150 und 290°C durchgeführt wird, wobei die in den Rohr- oder Autoklavenreaktor eingetragene Comonomerenmenge im Bereich von bis zu 60 Gew.-%, bezogen auf die gesamte in den Reaktor eingetragene Menge von Ethylen und Comonomeren, liegt.

5. Verfahren zur Herstellung eines Copolymers nach Anspruch 4, **dadurch gekennzeichnet, dass** die Copolymerisation von Schritt c) in einem Rohrreaktor durchgeführt wird, wobei die Mischung von Ethylen und Comonomeren mindestens an einem weiteren Punkt des Reaktors eingeleitet wird, wobei sich an diese Einleitung selbst eine erneute Einleitung von Initiator oder Initiatormischung anschließt.

## Claims

1. Process for the manufacture of a copolymer of ethylene and of at least one carboxylic acid vinyl ester comprising the following stages:
a) fermentation of renewable starting materials and optionally purification in order to produce at least one alcohol chosen from ethanol and mixtures of alcohols comprising ethanol;
b) dehydration of the alcohol obtained in order to produce at least one alkene chosen from ethylene and mixtures of alkenes comprising ethylene and optionally purification of the alkene in order to obtain ethylene,
c) copolymerization of the ethylene with at least one comonomer chosen from carboxylic acid vinyl esters,
d) isolation of the copolymer obtained.

2. Process for the manufacture of a terpolymer of ethylene, of at least one vinyl ester and of unsaturated carboxylic acid anhydride comprising the following stages:
a) fermentation of renewable starting materials and optionally purification in order to produce at least one alcohol chosen from ethanol and mixtures of alcohols comprising ethanol;
b) dehydration of the alcohol obtained in order to produce at least one alkene chosen from ethylene and mixtures of alkenes comprising ethylene and optionally purification of the alkene in order to obtain ethylene,
c) copolymerization of the ethylene with at least one vinyl ester and at least one unsaturated carboxylic acid anhydride,
d) isolation of the copolymer obtained.

3. Process for the manufacture of a copolymer according to Claim 1 or 2, **characterized in that** the renewable starting materials are plant materials chosen from sugar cane and sugar beet, maple, date palm, sugar palm, sorghum, century plant, maize, wheat, barley, rice, potato, cassava, sweet potato, algae or materials comprising cellulose or hemicellulose, such as wood, straw or paper.

4. Process for the manufacture of a copolymer according to any one of Claims 1 to 3, **characterized in that** the copolymerization of stage c) is a high pressure radical copolymerization carried out by introducing the ethylene, the comonomer or comonomers and an initiator of polymerization at high pressure either into a tubular reactor at a temperature of between 80 and 325°C or into an autoclave reactor at a temperature of between 150 and 290°C, the amount of the comonomers introduced into the tubular or autoclave reactor ranging up to 60% by weight, with respect to the total amount of ethylene and comonomers introduced into the reactor.

5. Process for the manufacture of a copolymer according to Claim 4, **characterized in that** the copolymerization of stage c) is carried out in a tubular reactor, the mixture of the ethylene and comonomers being injected at least at another point of the reactor, this injection being itself followed by a further injection of initiator or mixture of initiators.
